**Europäisches Patentamt**

**European Patent Office**

**Office européen des brevets**

(11) Veröffentlichungsnummer : **0 448 613 B1**

(12) **EUROPÄISCHE PATENTSCHRIFT**

(45) Veröffentlichungstag der Patentschrift :
05.08.92 Patentblatt 92/32

(51) Int. Cl.$^5$ : **C02F 3/00**, G01N 33/18

(21) Anmeldenummer : **90900834.4**

(22) Anmeldetag : **14.12.89**

(86) Internationale Anmeldenummer :
**PCT/EP89/01534**

(87) Internationale Veröffentlichungsnummer :
**WO 90/06900 28.06.90 Gazette 90/15**

(54) **VERFAHREN ZUM KONTINUIERLICHEN ÜBERWACHEN VON ABWASSER.**

(30) Priorität : **19.12.88 DE 3842734**

(43) Veröffentlichungstag der Anmeldung :
**02.10.91 Patentblatt 91/40**

(45) Bekanntmachung des Hinweises auf die
Patenterteilung :
**05.08.92 Patentblatt 92/32**

(84) Benannte Vertragsstaaten :
**AT BE CH DE FR GB IT LI NL SE**

(56) Entgegenhaltungen :
**EP-A- 0 009 580**
**WO-A-82/01419**
**DE-A- 2 728 071**

(73) Patentinhaber : **HOECHST
AKTIENGESELLSCHAFT
Postfach 80 03 20
W-6230 Frankfurt am Main 80 (DE)**

(72) Erfinder : **BAUER, Alexander
Lerchenweg 3
W-6233 Kelkheim (DE)**
Erfinder : **SELL, Günther
Stettiner Strasse 84
W-6234 Hattersheim am Main (DE)**

**Beschreibung**

Gegenstand der Erfindung ist ein Verfahren zum kontinuierlichen Überwachen von Abwasser auf abbaustörende, bakterienhemmende Wasserinhaltsstoffe durch Messen der Sauerstoffzehrung von mit dem Abwasser versetzter Bakterienschlammsuspension.

Bei der im allgemeinen großen Anzahl der an eine Kläranlage angeschlossenen Abwassereinleiter und bei der damit verbundenen Verschiedenartigkeit der Wasserinhaltsstoffe sind Effekte nicht auszuschließen, die durch Hemmstoffe im Abwasser hervorgerufen werden. Die wechselnde Zusammensetzung und Konzentration der Wasserinhaltsstoffe im Abwasserzulauf einer Kläranlage können unter Umständen auch zu einer Hemmung der Bakterientätigkeit, hervorgerufen durch synergistische Effekte, führen. Um die biologisch arbeitende Stufe der Kläranlage vor solchen abwasserbedingten Störungen zu schützen, müssen rechtzeitig Gegenmaßnahmen getroffen werden. Das erfordert aber eine entsprechende Abwasserkontrolle bereits im Vorfeld der Kläranlage. Hierfür werden sogenannte Toximeter verwendet, die intermittierend oder kontinuierlich mit sessilen oder suspendierten Bakterien arbeiten.

Nach DE 27 28 071 C2 ist ein Toximeterverfahren bekannt, bei dem zum Erkennen von bakterienschädigender Wasserinhaltsstoffe ein Gemisch aus Abwasser und Bakterienschlamm, der mit Sauerstoff angereichert ist, kontinuierlich einer Sauerstoffmeßstrecke zugeführt und durch Messen des Sauerstoffpartialdrucks am Anfang und Ende der Meßstrecke die Sauerstoffzehrung ermittelt wird.

Schwierigkeiten bereitet die Interpretation des gemessenen Sauerstoffverbrauchs. Auch wenn Einflußgrößen wie pH-Wert, Temperatur, Bakterienmasse und Abwassermenge konstant gehalten werden, ändert sich die Konzentration der Wasserinhaltsstoffe und damit die Sauerstoffzehrung ständig, so daß ein Rückgang des Sauerstoffverbrauchs entweder von einer verminderten Belastung oder einem Hemmeffekt herrühren kann. Um eine eindeutige Aussage zu erhalten, wird die Zufuhr von Abwasser unterbrochen und stattdessen Nährlösung dosiert, deren Sauerstoffbedarf bekannt ist. Wenn dabei auch eine verminderte Sauerstoffzehrung festgestellt wird, ist sicher, daß vom Abwasser ein Hemmeffekt ausgeht. Dieses Verfahren ist insbesondere bei häufig schwankender Abwasserbelastung lästig und sehr umständlich. hier will die Erfindung abhelfen.

Der Erfindung liegt demnach die Aufgabe zugrunde, ein Verfahren zu schaffen, mit dessen Hilfe sich die Sauerstoffzehrung unabhängig von der Abwasserbelastung eindeutig einer Hemmung der Bakterienaktivität zuordnen läßt.

Die Aufgabe wird durch ein Verfahren der eingangs beschriebenen Art gelöst, das dadurch gekennzeichnet ist, daß man einen Teilstrom des Abwassers einer Messung zuführt, mit der einer der Summenbelastungsparameter wie CSB, TOC, BSB ermittelt wird, einen zweiten Teilstrom des Abwassers mit sauerstoffreicher Bakterienschlammsuspension vermischt, das Gemisch in konstanter Menge einer Sauerstoffmeßstrecke zuführt, die Sauerstoffzehrung $\Delta pO_2$ mißt und aus der Sauerstoffzehrung und dem Wert des Summenbelastungsparameters die spezifische Sauerstoffzehrung $\Delta pO_2$ netto ermittelt.

Das Mengenverhältnis Abwasser zu Bakterienschlammsuspension kann innerhalb der Grenzen 1:5 bis 1:100 liegen. Der zweite Teilstrom des Abwassers soll möglichst nahe beim Eintritt in die Sauerstoffmeßstrecke mit dem Bakterienschlamm vermischt werden. Besonders gute Ergebnisse werden erzielt, wenn der Bakterienschlamm in gleichbleibender Konzentration eingesetzt wird, und zwar mit einem Trockensubstanzgehalt von 1 bis 10 g/l, einer Temperatur zwischen 10 und 45°C, einem pH-Wert von 5 bis 9 und eingangs der Meßstrecke einem Sauerstoffgehalt von >3 mg/l. Um ausreichende Mischung von Abwasser und Bakterienschlammsuspension zu gewährleisten, genügt es, wenn das Gemisch die Teststrecke mit 0,1 bis 5 m/min. durchströmt, sodaß die Verweilzeit in der Teststrecke dann 0,2 bis 10 min/m beträgt.

Die durch die Erfindung erreichten Vorteile sind im wesentlichen darin zu sehen, daß eine verminderte spezifische Sauerstoffzehrung weitgehend unabhängig vom Belastungsgrad des Abwassers ist und somit eindeutig einer Bakterienhemmung oder -schädigung zugeordnet werden kann.

Im folgenden wird die Erfindung anhand eines Fließschemas (Figur), das ein Ausführungsbeispiel darstellen soll, näher erläutert.

Dem Zulaufkanal der biologischen Kläranlage wird kontinuierlich Abwasser entnommen und über Leitung 9 der Apparatur für die Überwachung des Abwassers auf abbaustörende, bakterienhemmende Wasserinhaltsstoffe zugeführt. Diese Apparatur enthält u.a. ein Analysengerät 22 zum kontinuierlichen Messen eines der Summenbelastungsparameter Q, wie chemischer Sauerstoffbedarf (CSB), organisch gebundener Kohlenstoff (TOC) oder biologischer Sauerstoffbedarf (BSB), eine Meßstrecke, bestehend aus Meßsonden 18, 19 und Verweilzeitstrecke 20.

Die Meßstrecke 18, 19, 20 ist Teil eines Kreislaufs, bestehend aus einem Vorrats- und Aufbereitungsbekken 1 für den Bakterienschlamm, Pumpe 16, Rohrleitung 17, Meßstrecke 18, 19, 20 und Rohrleitung 21. Das Vorrats- und Aufbereitungsbecken 1 ist mit einem Nachklärteil versehen, das durch Trennwand 6 vom Belebungsteil abgetrennt ist und ein Überlaufwehr 7 aufweist. Das Analysengerät 22 und die Meßstrecke 18, 19, 20 sind mit einer Meßwertverarbeitungs- und Datenausgabeeinrichtung 23 verbunden (gestrichelt dargestellt), die die ermittelten Meßwerte 28, 29 ausgibt. Im Vorrats- und Aufbereitungsbecken 1 wird dem Bakterienschlamm über Leitung 4 und Gasverteilung 5 Sauerstoff und/oder Luft, über Leitung 3 Chemikalien zum Einstellen des pH-Wertes auf Werte von 5 bis 9 und über Leitung 2 eventuell ein Entschäumer und Nährsalzlösung zugegeben. Damit eine gute Durchmischung stattfindet ist im Becken 1 eine Trennwand 8, die Öffnungen 30 und 31 aufweist, angeordnet, um die die Suspension in einer Schlaufenbewegung kreist. Mit den Meßsonden 24, 23, 26, 27 werden pH-Wert, Trockensubstanzgehalt, Sauerstoffpartialdruck und Temperatur des Bakterienschlamms überwacht und innerhalb der vorerwähnten Grenzen konstant gehalten. Ein Teilstrom des Abwassers wird über Leitung 10 und Pumpe 13 dem Analysengerät 22 für die Messung des Summenbelastungsparameters Q zugeführt. Der im Analysengerät 22 gemessene Wert wird in der Meßwertverarbeitungs- und Datenausgabeeinrichtung 23 mit den Meßwerten aus der Meßstrecke 18, 19, 20 verarbeitet.

Ein zweiter Teilstrom des Abwassers wird über Leitung 11 und Pumpe 14 Leitung 17 zugeführt und dort vor Eintritt in die Meßstrecke mit dem Bakterienschlamm vermischt. Mit Sonde 18 wird der Sauerstoffpartialdruck am Eingang und mit Sonde 19 am Ausgang der Meßstrecke gemessen. Aus den Sauerstoffpartialdrücken, gemessen mit den Sonden 18, 19, ermittelt die Meßwertverarbeitungseinrichtung den Sauerstoffverbrauch $\Delta pO_2$ in der Meßstrecke 18, 19, 20 und unter Berücksichtigung der mit Sonde 26 gemessenen Grundzehrung den um die Grundzehrung verminderten Nettosauerstoffverbrauch $\Delta pO_2$ netto. Dieser Wert $\Delta pO_2$ netto wird in Relation zum Wert des Summenbelastungsparameters Q gesetzt und ergibt somit eine von Belastungsschwankungen weitgehend unabhängige, abwasserspezifische, dimensionslose Sauerstoffverbrauchszahl

$$\frac{\Delta pO_2 \text{ netto}}{Q}.$$

Die apparativen und meßtechnischen Einflüsse lassen sich an der Meßwertverarbeitungs- und Datenausgabeeinrichtung 23 durch Verstellen des Multiplikators kompensieren. Da die abwasserspezifische 02-Verbrauchszahl abhängig von den Wasserinhaltsstoffen in der Größenordnung um $\pm$ 20 % schwankt, wird die Empfindlichkeit zweckmäßig so gewählt, daß für den Normalfall die abwasserspezifische Sauerstoffverbrauchszahl bei ca. 70 % des Maximalausschlages (= 100 %) der Anzeige liegt. Der Rückgang der $O_2$-Verbrauchszahl auf weniger als die Hälfte des Normalwertes ist ein Zeichen für eine deutlich verstärkte Abbauhemmung der Bakterien und kann zum Auslösen von Alarmen an vorgewählten Schaltpunkten benutzt werden. So kann an einem ersten Schaltpunkt (zwischen 30 und 50 %) zunächst ein Voralarm gegeben werden, so daß Gelegenheit besteht, die Apparatur auf Konstanz der Parameter wie pH-Wert, Temperatur und Trockensubstanzgehalt zu überprüfen. Bei einem weiteren Rückgang der spezifischen Sauerstoffverbrauchszahl erfolgt an einem zweiten Schaltpunkt (<30 %) der eigentliche Alarm, durch den vorgesehene Präventivmaßnahmen in Gang gesetzt werden, z.B. Unterbrechung des Zulaufs zur Kläranlage. Reicht die Ernährung der Bakterien durch das durch Leitung 11 und Pumpe 13 zugeführte Abwasser nicht aus, kann weiteres Abwasser über Leitung 12 und Pumpe 15 der Bakterienschlammsuspension zugeführt werden. Das Verdrängungswasser wird über Nachklärteil und Wehr 7 dem Vorrats- und Aufbereitungsbecken 1 entnommen.

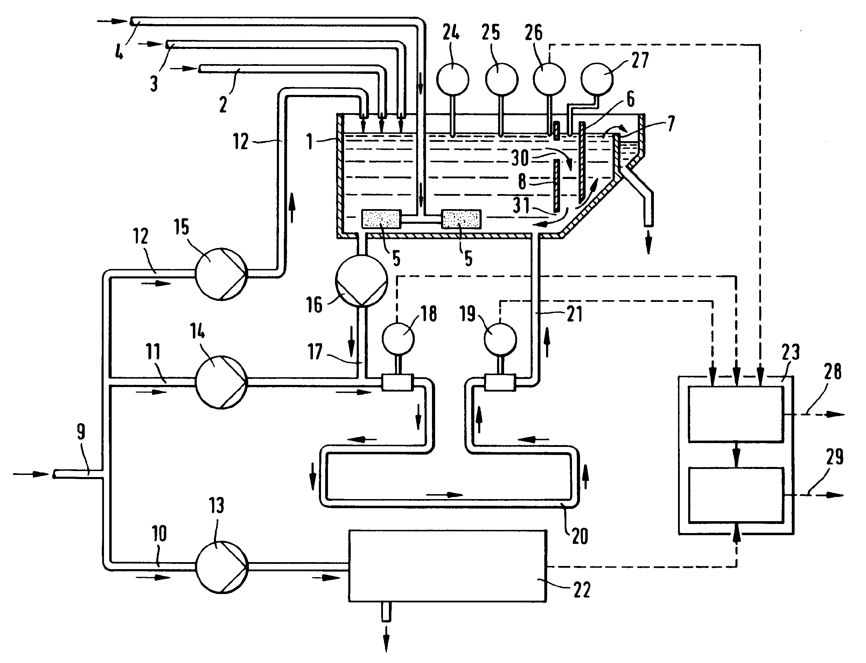

## Patentansprüche

1. Verfahren zum kontinuierlichen Überwachen von Abwasser auf abbaustörende, bakterienhemmende Wasserinhaltsstoffe durch Messen der Sauerstoffzehrung von mit dem Abwasser versetzter Bakterienschlammsuspension, dadurch gekennzeichnet, daß man einen Teilstrom des Abwassers einer Messung zuführt, mit der einer der Summenbelastungsparameter wie CSB, TOC oder BSB ermittelt wird, einen zweiten Teilstrom mit sauerstoffreicher Bakterienschlammsuspension vermischt, das Gemisch in konstanter Menge einer Sauerstoffmeßstrecke zuführt, die Sauerstoffzehrung $\Delta pO_2$ mißt und aus der Sauerstoffzehrung und dem Wert des Summenbelastungsparameters die spezifische Sauerstoffzehrung $\Delta pO_2$ netto ermittelt.

2. Verfahren nach Anspruch 1, dadurch gekennzeichnet, daß das Abwasser-Bakterienschlammgemisch mit konstantem Mengenverhältnis von Abwasser zu Bakterienschlammsuspension innerhalb der Grenzen 1:5 bis 1:100 der Sauerstoffmeßstrecke zugeführt wird, wobei der zweite Teilstrom des Abwassers bei Eintritt in die Meßstrecke mit dem Bakterienschlamm vermischt wird.

3. Verfahren nach Anspruch 1, dadurch gekennzeichnet, daß eine Bakterienschlammsuspension in gleichbleibender Konzentration eingesetzt wird, wobei die Suspension einen Trockensubstanzgehalt von 1 bis 10 g/l, eine Temperatur von 10 bis 45°C, einen pH-Wert von 5 bis 9 und eingangs der Meßstrecke einen Sauerstoffgehalt von $\geq$3 mg/l aufweisen soll.

## Claims

1. A method for continuous monitoring of effluent for water constituents which interfere with degradation and inhibit bacteria, by measuring the oxygen depletion of a bacteria sludge suspension with added effluent, which comprises passing a part-stream of the effluent to a measurement, by means of which one of the total pollution paramters such as COD, TOC or BOD is determined, mixing a second part-stream with oxygen-rich bacteria sludge suspension, feeding a constant rate of the mixture to an oxygen-measuring section, measuring the oxygen depletion $\Delta pO_2$ and determining the specific oxygen depletion $\Delta pO_2$ net from the oxygen depletion and the value of the total pollution parameter.

2. The method as claimed in claim 1, wherein the effluent/bacteria sludge mixture is fed at a constant effluent/bacteria sludge suspension rate ratio within the limits 1:5 to 1:100 to the oxygen-measuring section, the second part-stream of the effluent being mixed with the bacteria sludge on entering the measuring section.

3. The method as claimed in claim 1, wherein the bacteria sludge suspension is used at a constant concentration, the suspension being intended to have a dry matter content from 1 to 10 g/l, a temperature from 10 to 45°C, a pH from 5 to 9 and, at the entry to the measuring section, an oxygen content of $\geqq 3$ mg/l.

## Revendications

1. Procédé de surveillance continue des eaux résiduaires, pour le contrôle des composants de l'eau ayant un effet inhibiteur des bactéries et anti-dégradation, par mesure de la consommation d'oxygène par la suspension de boues bactériennes additionnée des eaux résiduaires, caractérisé en ce qu'on envoie un courant partiel des eaux résiduaires à un dispositif de mesure à l'aide duquel on détermine l'un des paramètres globaux de charge, comme la DCO, le carbone organique total TOC, la DBO ; on mélange un deuxième courant partiel des eaux résiduaires à une suspension de boues bactériennes riches en oxygrène ; on envoie le mélange, à débit constant, à une section de mesure de l'oxygène, on mesure la diminution de la pression d'oxygène $pO_2$ et, à partir de la diminution de la pression d'oxygène et de la valeur du paramètre global de charge, on détermine la valeur nette de la diminution spécifique de la pression d'oxygène $pO_2$.

2. Procédé selon la revendication 1, caractérisé en ce que le mélange des eaux résiduaires et des boues bactériennes est, pour un rapport pondéral constant des eaux résiduaires et de la suspension de boues bactériennes, compris entre 1:5 et 1:100, envoyé à la section de mesure de l'oxygène, le deuxième courant partiel des eaux résiduaires étant, au point d'entrée dans la section de mesure, mélangé aux boues bactériennes.

3. Procédé selon la revendication 1, caractérisé en ce qu'on utilise une suspension de boues bactériennes à une concentration constante, la suspension devant présenter une teneur en extrait sec de 1 à 10 g/l, une température de 10 à 45°C, un pH de 5 à 9 et, à l'entrée de la section de mesure, une teneur en oxygène $\geqq 3$ mg/l.

EP 0 448 613 B1